(19) Europäisches Patentamt
European Patent Office
Office européen des brevets

(11) Veröffentlichungsnummer: **0 082 401**
**B1**

(12) # EUROPÄISCHE PATENTSCHRIFT

(45) Veröffentlichungstag der Patentschrift: **03.07.85**

(51) Int. Cl.⁴: **C 07 D 309/04**

(21) Anmeldenummer: **82111294.3**

(22) Anmeldetag: **06.12.82**

(54) **Verfahren zur Herstellung von überwiegend das Z-Isomere enthaltendem Rosenoxid.**

(30) Priorität: **18.12.81 DE 3150234**

(43) Veröffentlichungstag der Anmeldung: **29.06.83 Patentblatt 83/26**

(45) Bekanntmachung des Hinweises auf die Patenterteilung: **03.07.85 Patentblatt 85/27**

(84) Benannte Vertragsstaaten: **CH DE FR GB LI NL**

(56) Entgegenhaltungen:
**WO - A - 79/00509**

**TETRAHEDRON LETTERS, Nr. 51, 1970, Seiten 4507-4508, Pergamon Press, London, G.B. J.H.P. TYMAN et al.: *"The reaction of 3-alkene-1-ols with aldehydes: A synthesis of (plusminus)-CIS-2-(2'methyl-1'-propenyl)-4-methyltetrahydropyran"***

(73) Patentinhaber: **BASF Aktiengesellschaft, Carl-Bosch-Strasse 38, D-6700 Ludwigshafen (DE)**

(72) Erfinder: **Hoffmann, Werner, Dr., Ringstrasse 11 C, D-6708 Neuhofen (DE)**

**EP 0 082 401 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein verbessertes Verfahren zur Herstellung eines Isomerengemisches von Z- und E-2-[2-Methyl-prop-1-en-1-yl]-4-methyl-tetrahydropyran, besser unter dem Namen Z- oder E-Rosenoxid bekannt, das mindestens 85% des für die Parfümerie wertvolleren natürlichen Z-Isomeren enthält.

Seit seiner Entdeckung im Jahre 1960 (vgl. CH 395.406) hat Rosenoxid steigendes Interesse in der Duftstoffindustrie gefunden. Das Z-Isomere hat sich als das wertvollere und daher begehrtere Isomere erwiesen. Es hat daher nicht an Versuchen gefehlt, Rosenoxid, insbesondere ein überwiegend das Z-Isomere enthaltendes Rosenoxid herzustellen. Die meisten bekannten Verfahren sehen die Verwendung von acyclischen Zwischenprodukten oder die Einführung des 2-Isobutylenrestes in den zunächst gebildeten Pyranring vor. So ist es beispielsweise aus Tetrahedron Letters No. *51* (1970) Seiten 4507-08 bekannt durch Umsetzen von 3-Methyl-2-buten-1-al und 2-Methyl-1-buten-4-ol und anschliessendes Dehydrieren das 2-[2-Methyl-prop-1-en-1-yl]-4-methylen-tetrahydropyran (II) herzustellen und dieses durch homogene Hydrierung in Gegenwart von $SnCl_2/H_2PtCl_6$ in ein hauptsächlich das Z-Isomere enthaltendes Rosenoxid zu überführen. Das genannte Verfahren ist für eine technische Anwendung jedoch noch nicht zufriedenstellend, da der für die Hydrierung verwendete Katalysator relativ teuer und ausserdem nur äusserst schwierig zurückzugewinnen ist, da das $H_2PtCl_6$ im Reaktionsprodukt löslich ist.

Eine Verbesserung dieses Verfahrens wird gemäss der PCT-Anmeldung mit der internationalen Publikationsnummer WO 79/00509 darin gesehen, II zu Rosenoxid zu reduzieren und anschliessend das noch grosse Anteile an dem E-Isomeren enthaltende Isomerengemisch durch Behandlung mit sauren Mitteln zu isomerisieren. Die Reduktion von II zu Rosenoxid erfolgt hierbei in Gegenwart von einem Pd-Katalysator oder von Raney-Nickel; die Isomerisierung in Gegenwart einer Protonensäure wie Schwefelsäure oder Phosphorsäure, einer sauren Tonerde oder einer sogenannten Lewis-Säure, insbesondere von $BF_3$ in Form seines Etherats. Nachteilig an diesem Verfahren ist, dass es sich bei der Hydrierung von II zu einem im wesentlichen Z-Rosenoxid enthaltendem Isomerengemisch praktisch um ein Zweistufenverfahren handelt, bei dem das zunächst erhaltene Isomerengemisch vor seiner Isomerisierung destillativ isoliert werden muss.

Es war daher die Aufgabe der Erfindung, ein Verfahren zu entwickeln, gemäss dem es gelingt, das -2-[2-Methyl-prop-1-en-yl]-4-methylen-tetrahydropyran II in Gegenwart von einem relativ leicht zugänglichen und ausserdem leicht aus dem Reaktionsgemisch abtrennbaren und somit leicht zurückgewinnbaren Katalysatorsystem in nur 1 Stufe zu einem im wesentlichen das Z-Isomere enthaltenden Rosenoxid zu gelangen.

Es wurde nun überraschenderweise gefunden, dass man II auf einfache Weise in einer Stufe zu einem mindestens 85% des Z-Isomeren enthaltenden Rosenoxids reduzieren kann, wenn man die Reduktion in Gegenwart von einem $PtO_2$- oder einem Pt/C-Katalysator in Gegenwart eines stark sauren Kationenaustauschers vornimmt.

Gegenstand der Anmeldung ist dementsprechend ein Verfahren zur Herstellung eines Isomerengemisches von Z- und E-2-[2-Methyl-prop-1-en-1-yl]-4-methyl-tetrahydropyran der Formeln Z-I und E-I

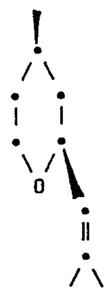
(Z-I)

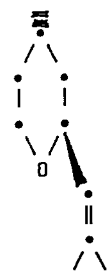
(E-I)

enthaltend mindestens 85% Z-I und höchstens 15% E-I, das dadurch gekennzeichnet ist, dass man 2-[2-Methyl-prop-1-en-1-1-yl]-4-methylen-tetrahydropyran der Formel II

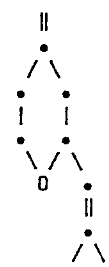
(II)

an einem Platindioxid- oder einem Platin/Kohle-Katalysator in Gegenwart eines stark sauren Kationenaustauschers hydriert.

Als Katalysatoren werden hierbei käufliche $PtO_2$-Katalysatoren bzw. Pt/C-Katalysatoren (0,5 bis 5%ig) eingesetzt.

Es war überraschend, dass das Verfahren mit so guten Ausbeuten gelingt, da bei entsprechender Hydrierung von II an $PtO_2$- oder Pt/C-Katalysatoren in Gegenwart anderer saurer Verbindungen entweder gar keine Hydrierung erfolgte oder aber zwar eine Hydrierung aber keine Isomerisierung stattfand (siehe Vergleichsbeispiele a bis f).

Als stark saure Kationenaustauscher verstehen wir erfindungsgemäss Kunstharz-Ionenaustauscher, d.h. hochpolymere räumliche Netzwerke aus Kohlenstoffketten (Matrix), die als ladungstragende Gruppen (Festionen) $-SO_3^{\ominus}$-Gruppen oder $-SO_3^{\ominus}$- und $-O^{\ominus}$-Gruppen enthalten. Es handelt sich hierbei im wesentlichen um handelsübliche Kationenaustauscher auf der Basis von Polystyrolsulfonsäureharzen oder Phenolsulfonsäureharzen, die z.B. unter folgenden Handelsnamen bekannt sind: Lewatit S 100, Lewatit S 115, Lewatit SP 1080, Lewatit SC 102, Lewatit SPC 118, Amberlite IR 120, Amberlite IR 200, Amberlyst 15, Dowex 50, Permutit RS, Wofatit KPS 200, Duolite

C-3, Duolite C-10, Duolite C-25, Wofatit F, Wofatit D, Wofatit P, Zeoxex (Zeokarb H), Nalcite HCR, Nalcite HGR, Nalcite HDR, Permutit Q und Permutit RS.

Bezüglich näherer Einzelheiten über Herstellung, Eigenschaften und Verwendung der sauren Kationenaustauscher verweisen wir auf Ullmanns Encyclopädie der technischen Chemie, 3. Auflage, Band 8, 1957, Seite 787 ff, insbesondere Seite 806 bis 811 und 814 bis 822.

Besonders geeignet sind stark saure Ionenaustauscher, die darüber hinaus makroporös sind wie z.B. Lewatit SPC 118/H oder Amberlite 200. Die Austauscher wurden in trockener $H^{\oplus}$-Form eingesetzt.

Bei diskontinuierlicher Arbeitsweise werden im allgemeinen zwischen 0,5 und 1 g des Hydrierkatalysators sowie zwischen 10 und 100 ml des Ionenaustauschers pro Mol II eingesetzt. Eine besondere Ausführungsform des Verfahrens ist jedoch die Anordnung von Hydrierkatalysator und Ionenaustauscher im Festbett, wobei wesentlich geringere Mengen benötigt werden.

Die Umsetzung kann sowohl lösungsmittelfrei als auch in einem inerten organischen Lösungsmittel, wie Methyl-tert.-butylether, Diethylether, Tetrahydrofuran oder Cyclohexan durchgeführt werden.

Die Reaktionstemperaturen betragen im allgemeinen 20 bis 50° C, vorzugweise Raumtemperatur.

Der Wasserstoffüberdruck während der Hydrierung liegt im allgemeinen zwischen 0 und 1 bar.

Das als Ausgangsverbindung für das erfindungsgemässe Verfahren benötigte Dehydrorosenoxid II kann leicht gemäss dem in Tetrahedron Letters *51* (1970), Seite 4507 beschriebenen Verfahren hergestellt werden. Die hierfür einzusetzenden Ausgangsstoffe, das 3-Methyl-2-buten-1-al sowie das 2-Methyl-1-buten-4-ol sind handelsübliche Produkte.

Das Verfahrensprodukt kann in einfacher Weise durch Abfiltrieren des Katalysatorsystems und anschliessende Destillation isoliert werden.

Sowohl die Lösungsmittel als auch das Katalysatorsystem können erneut verwendet werden.

Das Verfahrensprodukt ist ein besonders wertvoller, auch in der Natur vorkommender Duftstoff.

*Beispiel 1*

1216 g (~ 8 Mol) eines etwa 97%igen Dehydrorosenoxids wurden in 1 l Methyl-tert.-butylether aufgenommen, mit 1 g $PtO_2$ und 250 ml Lewatit SPC 118 (Lewatit-Ionenaustauscher) in trockener $H^{\oplus}$-Form versetzt und bei 25° C und 0,3 bar Wasserstoffdruck hydriert. Die Hydrierung wurde nach Aufnahme von 192 l Wasserstoff abgebrochen, wozu etwa 11 Stunden notwendig waren. Der Reaktionsverlauf wurde durch stündlich durchgeführte gaschromatographische Analysen (GC) verfolgt. Anschliessend wurde vom Ionenaustauscher und Katalysator abfiltriert, das Lösungsmittel bei 20° C und 20 mbar abdestilliert und das verbleibende Hydrierungsprodukt einer fraktionierten Destillation unterworfen. Man erhielt 1015 g eines Produkts vom Siedepunkt Kp = 89 bis 90° C (26 mbar), das nach GC 92% Z-Rosenoxid und 6,5% E-Rosenoxid enthielt. Das entspricht einer Ausbeute von 82% der Theorie. $n_D^{25}$ = 1,4536.

*Beispiel 2*

152 g (1 Mol) eines etwa 97%igen Dehydrorosenoxids wurden mit 0,5 g Platin/Kohle (5%ig) und 50 ml Ionenaustauscher Lewatit SPC 118 ($H^{\oplus}$-Form, trocken) versetzt und bei 25 bis 30° C und 0,3 bar Wasserstoffdruck hydriert. Die Hydrierung wurde nach 26 Stunden, in denen 23,5 l Wasserstoff aufgenommen wurde, abgebrochen. Der Reaktionsverlauf wurde durch GC verfolgt. Man filtrierte dann vom Katalysator und Ionenaustauscher ab, wusch anhaftendes Produkt mit Diethylether aus, destillierte das Lösungsmittel bei 50° C und 20 mbar ab und fraktionierte das zurückbleibende Produkt über eine 1 m Kolonne, die mit 5 mm Glasringen gefüllt war. Man erhielt 129 g eines Produkts, das nach GC 90,5% Z-Rosenoxid und 7% E-Rosenoxid enthielt. Das entspricht einer Ausbeute von 87% der Theorie.

*Vergleichsbeispiele*

a) 152 g (1 Mol) eines 97%igen Dehydrorosenoxids wurden mit 0,5 g Platin/Kohle (5%ig) und 1 g Zinn(II)chlorid versetzt und unter kräftigem Rühren bei 25° C einer Wasserstoffatmosphäre (Normaldruck bis 0,3 bar) ausgesetzt. In 24 Stunden erfolgte keine Wasserstoffaufnahme.

b) Der unter a) beschriebene Versuch wurde unter Verwendung von 1 ml Borfluoridetherat anstelle von 1 g $SnCl_2$ wiederholt. In 24 Stunden erfolgte ebenfalls keine Wasserstoffaufnahme.

c) Der unter a) beschriebene Versuch wurde unter Verwendung von 1 g p-Toluolsulfonsäure anstelle von 1 g $SnCl_2$ wiederholt. Nach 51 Stunden waren etwa 24 l $H_2$ aufgenommen. Dann wurde die Hydrierung unterbrochen, vom Katalysator abfiltriert, mit $NaHCO_3$-Lösung neutral gewaschen und destilliert. Es wurden 143 g Destillat erhalten, das nach GC- und NMR-Analyse 30% Z-Rosenoxid und 52% E-Rosenoxid enthält.

d) 152 g (1 Mol) eines etwa 97%igen Dehydrorosenoxids wurden mit 0,5 g $PtO_2$ und 2 g $SnCl_2$ versetzt und unter kräftigem Rühren bei 25 bis 30° C und 0,3 bar Wasserstoffdruck versucht zu hydrieren. In 24 Stunden erfolgte keine Wasserstoffaufnahme.

e) Der unter d) beschriebene Versuch wurde unter Verwendung von 2 ml Borfluoridetherat anstelle von 2 g $SnCl_2$ wiederholt. In 4 Stunden wurden bei 25 bis 30° C 24 l Wasserstoff aufgenommen. Die Aufarbeitung erfolgte analog Versuch c). Bei der Destillation wurden 144 g Produkt erhalten, das nach GC- und NMR-Analyse 25% Z-Rosenoxid und 58% E-Rosenoxid enthielt.

f) Der unter d) beschriebene Versuch wurde unter Verwendung von 2 g p-Toluolsulfonsäure anstelle von 2 g $SnCl_2$ wiederholt. Bei 25 bis 30° C war die Wasserstoffaufnahme von 24 l innerhalb von 3 Stunden beendet. Da das Isomerenverhältnis zu diesem Zeitpunkt nach GC-Analyse bei 31%

Z- und 55% E-Rosenoxid lag, wurde weitere 24 Stunden bei 25 bis 30° C nachgerührt. Anschliessend wurde gemäss Versuch 3 aufgearbeitet. Bei der Destillation fielen 144 g Produkt an, das nach GC-Analyse 32% Z- und 54% E-Rosenoxid enthielt. Eine Isomerisierung in Richtung Z-Rosenoxid fand praktisch nicht statt.

## Patentanspruch

Verfahren zur Herstellung eines Isomerengemisches von Z- und E-2-[2-Methyl-prop-1-en-yl]-4-methyl-tetrahydropyran der Formeln Z-I und E-I

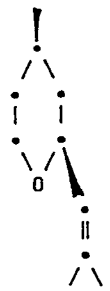
(Z-I)

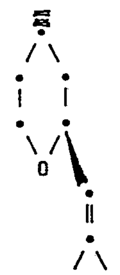
(E-I)

enthaltend mindestens 85% Z-I und höchstens 15% E-I, dadurch gekennzeichnet, dass man 2-[2-Methyl-prop-1-en-1-yl]-4-methylen-tetrahydropyran der Formel II

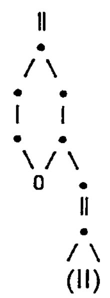
(II)

an einem Platindioxid- oder einem Platin/Kohle-Katalysator in Gegenwart eines stark sauren Kationenaustauschers hydriert.

## Claim

A process for the preparation of a mixture of Z- and E-2-[2-methylprop-1-en-1-yl]-4-methyltetrahydropyran isomers of the formulae Z-I and E-I

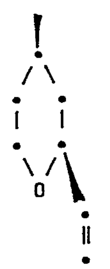
(Z-I)

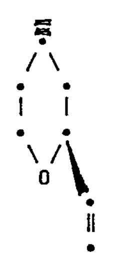
(E-I)

containing at least 85% of Z-I and not more than 15% of E-I, wherein 2-[2-methylprop-1-en-1-yl]-4-methylenetetrahydropyran of the formula II

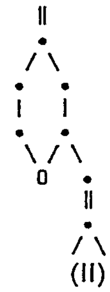
(II)

is hydrogenated over a platinum dioxide or platinum/charcoal catalyst in the presence of a strongly acidic cation exchanger.

## Revendication

Procédé pour la préparation d'un mélange d'isomères Z et E du 2-[2-méthyl-prop-1-ène-1-yl]-4-méthyl-tétrahydropyranne de formules Z-I et E-I

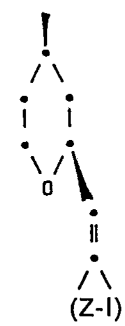
(Z-I)

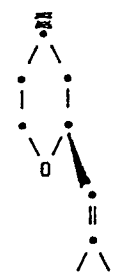
(E-I)

contenant au moins 85% de Z-I et au maximum 15% de E-I, caractérisé en ce qu'on hydrogène le 2-[2-méthyl-prop-1-ène-1-yl]-4-méthylène-tétrahydropyranne de formule II

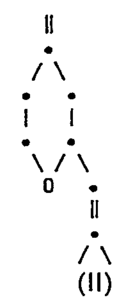
(II)

sur un catalyseur d'oxyde platinique ou de platine/charbon en présence d'un échangeur de cations fortement acide.